# EUROPEAN PATENT APPLICATION

(11) **EP 4 036 107 A1**
(43) Date of publication of application: **03.08.2022**
(21) Application number: 19947045.1
(22) Date of filing: 30.09.2019
(51) Int. Cl.: C07K 14/35, C12N 15/31, C12N 15/63, C12Q 1/68, G01N 33/68

(54) **MMUP MONOMER VARIANT AND APPLICATION THEREOF**

(30) Priority: 29.09.2019 CN 201910936950
(71) Applicant: Qitan Technology Ltd., Beijing, Beijing 100192 (CN)
(72) Inventor: LIU, Shaowei, Beijing 100192 (CN); ZHOU, Ya, Beijing 100192 (CN); CHEN, Chengyao, Beijing 100192 (CN)
(74) Representative: HGF
(86) International application number: PCT/CN2019/109332
(87) International publication number: WO 2021/056598

(57) **Abstract**

The invention provides a Mmup monomer variant comprising an amino acid sequence with any one or more amino acid mutations at 91st to 99th sites of SEQ ID NO: 1, as well as a porin or a construct comprising at least one Mmup monomer variant and application of the porin or the construct. The invention also provides a method of characterizing a target polynucleotide.

## Description

### Technical field

The invention relates to the technical field of characterization of nucleic acid characteristics, in particular to a Mmup monomer variant, a porin and a construct comprising the Mmup monomer variant, and a method for characterizing a target polynucleotide by using the Mmup monomer variant or the porin.

### Background technique

Nanopore sequencing technology is a gene sequencing technology which takes a single-stranded nucleic acid molecule as a sequencing unit, utilizes a nanopore providing an ion current channel and makes the single-stranded nucleic acid molecules pass through the nanopore under the force of an electric field. When the polynucleotide passes through the nanopore to translocate, the corresponding blocking current is generated due to the physical space-occupying effect, and the different signals generated are read in real time and analyzed to obtain the polynucleotide sequence information. Nanopore sequencing technology has the following advantages: A library can be easily builded without amplification; the reading speed is fast, and the reading speed of single-stranded molecules can reach tens of thousands of bases per hour; the reading length is longer and can reach thousands of bases generally; and methylated DNA or RNA can be directly measured.

However, when each nucleotide or a series of nucleotides pass through the nanoporin in the effect of electric field force, one specific blocking current is generated. Although the current signal recorded at this time corresponds to the polynucleotide sequence, it is generally 3 to 4 nucleotides that control current levels at certain grades, so the accuracy still needs to be improved. Currently, the accuracy can be improved by changing the structure of the polynucleotide, the duration at the nanopore, and developing the new nanopores to control the translocation of the polynucleotide.

For example: Patent US20150065354A1 discloses a method of characterizing a target polynucleotide by using a XPD helicase, the method uses a pore and a XPD helicase. The XPD helicase of the invention can control the movement of the target polynucleotide through the pore.

Patent US20170268055A1 discloses a composition and method for polynucleotide sequencing. The method utilizes the translocation steps of the target polynucleotide through pore to characterize the target polynucleotide, including the method and the composition of characterizing the polynucleotide sequence.

Patent CN106103741A discloses a method of linking one or more polynucleotide binding proteins to the target polynucleotide, and the invention also relates to a new method of characterizing the target polynucleotide.

Patent CN102216783B discloses a *Mycobacterium smegmatis* porin (Msp) nanopore and uses the Msp nanopore for sequencing, wherein the amino acid at 90th or 91st site of the wild-type Msp is mutated to improve the conductance of the analyte in sequencing, and reduce the translocation speed of analytes in sequencing.

However, the current technology does not mention the Mmup monomer variants and their applications in sequencing, and there are few types of porins that can be used for sequencing in the current technology.

Therefore, the invention further provides a new nanoporin. The Mmup monomer variants are prepared by mutating the wild-type Mmup proteins that can not be used for sequencing, and confirmed the function of the Mmup monomer variants in sequencing.

### The invention contents

The invention proves that the preparation of the Mmup monomer variants with site-specific mutations of the Mmup mutant protein can be used for nanopore sequencing, but the wild-type Mmup monomer does not have the function. And by applying the porins of the invention herein to nanopore sequencing, the difference in current signals of various nucleotides can be clearly observed, the sequencing accuracy is higher.

The "Mmup" in the present invention herein is derived from *Mycobacterium mucinae.* Preferably, the "Mmup" is derived from *Mycolicibacterium mucogenicum.*

Specifically, the first aspect of the present invention provides a Mmup monomer variant, the Mmup monomer variant comprises an amino acid sequence with any one or more amino acid mutations at 91st to 99th sites of SEQ ID NO: 1.

Preferably, the Mmup monomer variant comprises the mutation of aspartic (D) at 91st site and/or alanine (A) at 99th site.

In a specific embodiment of the present invention, the Mmup monomer variant comprises the mutation of aspartic (D) at 91st site.

In another specific embodiment of the present invention, the Mmup monomer variant comprises the mutation of alanine (A) at 99th site.

In another specific embodiment of the present invention, the Mmup monomer variant comprises the mutations of aspartic (D) at 91st site and alanine (A) at 99th site.

Preferably, the Mmup monomer variant comprises at least one of the following mutations:
Aspartic (D) at 91st site is mutated to: proline (P), tryptophan (W), arginine (R), glutamine (Q), lysine (K), phenylalanine (F), serine (S ), asparagine (N), cysteine (C), isoleucine (I), leucine (L) or valine(V), or non-natural amino acid; or,
Alanine (A) at 99th site is mutated to: proline (P), phenylalanine (F), isoleucine (I), leucine (L), valine (V), lysine(K) or arginine (R), or, non-natural amino acid.

Further Preferably, the Mmup monomer variant comprises mutations of D91K and/or A99K.

In a specific embodiment of the present invention, the Mmup monomer variant comprises the mutation of D91K.

In another specific embodiment of the present invention, the Mmup monomer variant comprises the mutation of A99K.

In another embodiment of the present invention, the Mmup monomer variant comprises the mutations of D91K and A99K.

Preferably, the Mmup monomer variant also comprises an amino acid sequence with any one or more amino acid mutations at 80th to 90th sites and/or 100th to 120th sites of SEQ ID NO: 1.

Further preferably, the Mmup monomer variant also comprises an amino acid sequence with any one or more amino acid mutations at 1st to 79th sites and/or 121st to 186th sites of SEQ IDNO: 1.

Preferably, the Mmup monomer variant also comprises one or a combination of two or more of the mutation of leucine (L) at 89th site, the mutation of asparagine (N) at 110th site, the mutation of aspartic (D) at 120th site, the mutation of asparagine (N) at 136th site or the mutation of serine (S) at 141st site.

Further preferably, the Mmup monomer variant comprises at least one of the following mutations:
Leucine (L) at 89th site is mutated to: asparagine (N), alanine (A), glutamine (Q), glycine (G), serine (S), threonine (T), lysine (K) or proline (P), or, non-natural amino acid; or,
Asparagine (N) at 110th site is mutated to: proline (P), alanine (A), isoleucine (I) or leucine (L), or non-natural amino acid; or,
Aspartic (D) at 120th site is mutated to: arginine (R), lysine (K), asparagine (N), glutamine (Q), alanine (A), serine (S), glycine (G) or threonine(T), or non-natural amino acid; or,
Asparagine (N) at 136th site is mutated to: arginine (R), lysine (K), glutamine (Q), alanine (A), serine (S) or threonine (T), or non-natural amino acid; or,
Serine (S) at 141st site is mutated to: arginine (R), lysine (K), glutamine (Q), alanine (A), serine (S) or threonine (T), or non-natural amino acid.

Preferably, the Mmup monomer variant also comprises other mutation types in addition to the mutation types as described above, as long as the mutation does not affect the differentiation of different polynucleotides when the polynucleotide passes through the porin.

Preferably, the Mmup monomer variant also comprises a mutation that introduces cysteine in order to link with the molecules for sequencing, such as the nucleic acid binding protein.

Preferably, the Mmup monomer variant can only comprise the constriction and loops region sequence fragments of the porin forming domain, and retains the activity to form pores. The redundant residues can be removed or other amino acid residues can be added and the activity to form pores is retained. The fragment length can be at least 12, 20, 40, 50, 100 or 150 amino acids.

Preferably, the Mmup monomer variant can be modified to facilitate identification or purification. For example: by adding aspartic residues (asp tag), streptavidin tag, flag tag or histidine residue (His tag).

Preferably, the Mmup monomer variant can carry with a display marker. For example: fluorescent molecules, radioisotope ¹²⁵I, radioisotope ³⁵S, polynucleotide, biotin, antigen or antibody.

Preferably, the Mmup monomer variant also comprises a molecular engine. Preferably, the molecular engine is an enzyme. Further preferably, the enzyme is polymerase, exonuclease or Klenow fragment.

The second aspect of the present invention provides a construct comprising at least one of any one Mmup monomer variant described in the present invention. Wherein, the construct retains the ability to form pores.

Preferably, the construct comprises 1 to 50 Mmup monomer variants, wherein, the Mmup monomer variants are identical or different. Specifically, the construct comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 Mmup monomer variants, wherein the Mmup monomer variants are identical or different.

Further preferably, the construct comprises 1 to 20 Mmup monomer variants, wherein the Mmup monomer variants are identical or different. Specifically, the construct comprises 1, 2, 3, 4, 5, 6, 7, 8, 9, 10, 11, 12, 13, 14, 15, 16, 17, 18, 19 or 20 Mmup monomer variants, wherein the Mmup monomer variants are identical or different.

Preferably, the construct also comprises the wild-type Mmup monomer.

Further preferably, the construct comprises 1 to 50 wild-type Mmup monomers. Specifically, the construct comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 wild-type Mmup monomers.

In a specific embodiment of the present invention, the construct comprises 1 to 20 wild-type Mmup monomers. Specifically, the construct comprises 1, 2, 3, 4, 5, 6, 7, 8, 9, 10, 11, 12, 13, 14, 15, 16, 17, 18, 19 or 20 wild-type Mmup monomers.

Most preferably, the construct comprises 4 to 10 identical or different Mmup monomer variants.

In a specific embodiment of the present invention, the construct comprises 4, 6, 8, 10 identical or different Mmup monomer variants.

Preferably, the Mmup monomer variant and the Mmup monomer variant, the wild-type Mmup monomer and the wild-type Mmup monomer, as well as the Mmup monomer variant and the wild-type Mmup monomer are covalently attached.

Preferably, the Mmup monomer variant and the Mmup monomer variant, the wild-type Mmup monomer and the wild-type Mmup monomer, as well as the Mmup monomer variant and the wild-type Mmup monomer are genetically fused.

The third aspect of the present invention provides a porin comprising at least one Mmup monomer variant, the Mmup monomer variant comprises the amino acid sequence with any one or more amino acid mutations at 91st to 99th sites of SEQ ID NO: 1, the mutations lead to make a difference in electrical resistance within the pore due to the difference in the physical or chemical properties of different kinds of nucleotides when the single polynucleotide strand passes through the porin comprising at least one Mmup monomer variant.

Preferably, the mutations lead to a change in charge properties or hydrophobic properties of amino acids.

Preferably, the difference in electrical resistance refers to the characteristics that can be used to characterize the polynucleotide, and the characteristics comprise the source, length, size, molecular weight, identity, sequence, secondary structure, concentration of the polynucleotide, or whether the target polynucleotide is modified. Further preferably, the difference in electrical resistance refers to the sequence characteristics that can be used to characterize the polynucleotide, that is, the porin can be used for sequencing and accurately distinguishing different bases of the polynucleotide.

Preferably, the polynucleotide can be natural or artificially synthesized. Further preferably, the polynucleotide can be natural DNA, RNA or modified DNA or RNA.

Still further preferably, one or more nucleotides of the target polynucleotide can be modified, such as methylation, oxidation, damage, abasic, protein labeling, with tagging or a spacer linked in the middle of the polynucleoside sequence.

Still further preferably, the artificially synthesized nucleic acid is selected from peptide nucleic acid (PNA), glycerol nucleic acid (GNA), threose nucleic acid (TNA), locked nucleic acid (LNA) or other synthetic polymers with nucleoside side chains.

Preferably, the target polynucleotide is single-stranded, double-stranded, or at least a part of the target polynucleotide is double-stranded.

Preferably, the Mmup monomer variant comprises the mutations of aspartic (D) at 91st site and/or alanine (A) at 99th site.

In a specific embodiment of the present invention, the Mmup monomer variant comprises the mutation of aspartic (D) at 91st site.

In another specific embodiment of the present invention, the Mmup monomer variant comprises the mutation of alanine (A) at 99th site.

In another specific embodiment of the present invention, the Mmup monomer variant comprises the mutations of aspartic (D) at 91st site and alanine (A) at 99th site.

Further preferably, the Mmup monomer variant comprises at least one of the following mutations:
Aspartic (D) at 91st site is mutated to: proline (P), tryptophan (W), arginine (R), glutamine (Q), lysine (K), phenylalanine (F),serine (S ), asparagine (N), cysteine (C), isoleucine (I), leucine (L) or valine (V), or non-natural amino acid;or,
Alanine (A) at 99th site is mutated to: proline (P), phenylalanine (F), isoleucine (I), leucine (L), valine (V), lysine (K) orarginine (R), or, non-natural amino acid.

Still further preferably, the Mmup monomer variant comprises the mutations of D91K and/or A99K.

In a specific embodiment of the present invention, the Mmup monomer variant comprises the mutations of D91K and A99K.

Preferably, the Mmup monomer variant also comprises an amino acid sequence with any one or more amino acid mutations at 80th to 90th sites and/or 100th to 120th sites of SEQ ID NO: 1.

Further preferably, the Mmup monomer variant also comprises an amino acid sequence with any one or more amino acid mutations at 1st to79th sites and/or 121st to 186th sites of SEQ ID NO: 1.

Preferably, the Mmup monomer variant also comprises one or a combination of two or more of the mutation of leucine (L) at 89th site, the mutation of asparagine (N) at 110th site, the mutation of aspartic (D) at 120th site, the mutation of asparagine (N) at 136th site or the mutation of serine (S) at 141st site.

Further preferably, the Mmup monomer variant comprises at least one of the following mutations:
Leucine (L) at 89th site is mutated to: asparagine (N), alanine (A), glutamine (Q), glycine (G), serine (S), threonine (T), lysine (K) or proline (P), or, non-natural amino acid; or,
Asparagine (N) at 110th site is mutated to: proline (P), alanine (A), isoleucine (I) or leucine (L), or non-natural amino acid; or,
Aspartic (D) at 120th site is mutated to: arginine (R), lysine (K), asparagine (N), glutamine (Q), alanine (A), serine (S), glycine (G) or threonine(T), or non-natural amino acid; or,
Asparagine (N) at 136th site is mutated to: arginine (R), lysine (K), glutamine (Q), alanine (A), serine (S) or threonine (T), or non-natural amino acid; or,
Serine (S) at 141st site is mutated to: arginine (R), lysine (K), glutamine (Q), alanine (A), serine (S) or threonine (T), or non-natural amino acid.

Preferably, the porin comprising at least one Mmup monomer variant comprises at least 1 to 50 Mmup monomer variants, wherein the Mmup monomer variants are identicalor different.

Specifically, the porin comprising at least one Mmup monomer variant comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 Mmup monomer variants, wherein the Mmup monomer variants are identical or different.

Further preferably, the porin comprising at least one Mmup monomer variant comprises at least 1 to 20 Mmup monomer variants, wherein the Mmup monomer variants are identical or different.

Specifically, the porin comprising at least one Mmup monomer variant comprises 1, 2, 3, 4, 5, 6, 7, 8, 9, 10, 11, 12, 13, 14, 15, 16, 17, 18, 19 or 20 Mmup monomer variants, the Mmup monomer variants are identical or different.

Preferably, the porin comprising at least one Mmup monomer variant also comprises the wild-type Mmup monomer.

Preferably, the porin comprising at least one Mmup monomer variant comprises 1 to 50 wild-type Mmup monomers.

Specifically, the porin comprising at least one Mmup monomer variant comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 wild-type Mmup monomers.

Further preferably, the porin comprising at least one Mmup monomer variant comprises 1 to 20 wild-type Mmup monomers.

Specifically, the porin comprising at least one Mmup monomer variant comprises 1, 2, 3, 4, 5, 6, 7, 8, 9, 10, 11, 12, 13, 14, 15, 16, 17, 18, 19 or 20 wild-type Mmup monomers.

In a specific embodiment of the present invention, the porin comprising at least one Mmup monomer variant comprises 4 to 10 identical or different Mmup monomer variants.

In a specific embodiment of the present invention, the porin comprising at least one Mmup monomer variant comprises 4, 6, 8, 10 identical or different Mmup monomer variants.

Preferably, the Mmup monomer variant and the Mmup monomer variant, the wild-type Mmup monomer and the wild-type Mmup monomer, as well as the Mmup monomer variant and the wild-type Mmup monomer are covalently attached.

Preferably, the Mmup monomer variant and the Mmup monomer variant, the wild-type Mmup monomer and the wild-type Mmup monomer, as well as the Mmup monomer variant and the wild-type Mmup monomer are genetically fused.

Preferably, the Mmup monomer variants comprised in the porin comprising at least one Mmup monomer variant are identical or different. For example, the porin comprising at least one Mmup monomer variant can comprise eight identical or different Mmup monomer variants. Preferably, the porin comprising at least one Mmup monomer variant comprises one Mmup monomer variant and seven identical monomers, wherein the Mmup monomer variant is different from identical monomer.Or, the porin comprising at least one Mmup monomer variant comprises two identical or different Mmup monomer variants and six identical monomers, wherein the Mmup monomer variants are different from identical monomer. Or, the porin comprising at least one Mmup monomer variant comprises three identical or different Mmup monomer variants and five identical monomers, wherein the Mmup monomer variants are different from identical monomer. Or, the porin comprising at least one Mmup monomer variant comprises four identical or different Mmup monomer variants and four identical monomers, wherein the Mmup monomer variants are different from identical monomer. Or, the porin comprising at least one Mmup monomer variant comprises five identical or different Mmup monomer variants and three identical monomers, wherein the Mmup monomer variants are different from identical monomer. Or, the porin comprising at least one Mmup monomer variant comprises six identical or different Mmup monomer variants and two identical monomers, wherein the Mmup monomer variants are different from identical monomer. Or, the porin comprising at least one Mmup monomer variant comprises seven identical or different Mmup monomer variants and one monomer, wherein the Mmup monomer variants are different from one monomer.

Preferably, the porin comprising at least one Mmup monomer variant comprises eight identical or different Mmup monomer variants.

Preferably, the porin comprising at least one Mmup monomer variant can be homologous or heterologous.

Preferably, the porin comprising at least one Mmup monomer variant comprises the constriction and the loops region.

Preferably, the pore channel diameter of the constriction of the porin comprising at least one Mmup monomer variant is less than the pore channel diameter of the constriction of the porin comprising the wild-type Mmup monomer. Further preferably, the pore channel diameter of the constriction of the porin comprising at least one Mmup monomer variant is less than 15.5Å, 15.4Å, 15.3Å, 15.2Å, 15.1Å, 15.0Å, 14.9Å, 14.8Å, 14.7Å, 14.6Å, 14.5Å, 14.4Å, 14.3Å, 14.2Å, 14.1Å, 14.0Å, 13.9Å, 13.8Å, 13.7Å, 13.6Å, 13.5Å, 13.4Å or 13.3Å; Still further preferably, the pore channel diameter of the constriction of the porin comprising at least one Mmup monomer variant is less than 13.5Å, 13.4Å, 13.3Å, 13.2Å, 13.1Å, 13.0Å, 12.9Å, 12.8Å, 12.7Å, 12.6Å or 12.5Å_{∘}

In a specific embodiment of the present invention, the pore channel diameter of the constriction of the porin comprising at least one Mmup monomer variant is approximately equal to 13.3Å or 13.0Å_{∘}

Preferably, the Mmup monomer variant can also comprise other mutation types in addition to the mutation types as described above, as long as the mutation does not affect the differentiation of different polynucleotides when the polynucleotide passes through the porin.

Preferably, the Mmup monomer variant canalso comprise a mutation that introduces cysteine in order to link with the molecules for sequencing, such as the nucleic acid binding protein.

Preferably, the Mmup monomer variant can only comprise the constriction and the loops region sequence fragments of the porin forming domain, and retains the activity to form pores. The redundant residues can be removed or other amino acid residues can be added and the activity to form pores is retained. The fragment length can be at least 12, 20, 40, 50, 100 or 150 amino acids.

Preferably, the Mmup monomer variant can be modified to facilitate identification or purification. For example: by adding aspartic residues (asp tag), streptavidin tag, flag tag or histidine residue (His tag).

Preferably, the Mmup monomer variant can carry with a display marker. For example: fluorescent molecules, radioisotope ¹²⁵I, radioisotope ³⁵S, polynucleotide, biotin, antigen or antibody.

Preferably, the Mmup monomer variant also comprises a molecular engine.

Preferably, the molecular engine is an enzyme. Further preferably, the enzyme is polymerase, exonuclease or Klenow fragment.

Preferably, the porin comprising at least one Mmup monomer variant also comprises a cap forming region and/or a barrel forming region.

Preferably, the porin comprising at least one Mmup monomer variant allows hydrated ions to flow from one side of the membrane to the other layer of the membrane under the driving of an applied electric potential. Wherein, the membrane is a double-layer membrane, further preferably a lipid bilayer membrane.

The fourth aspect of the present invention provides a nucleotide sequence encoding any one of porins comprising at least one Mmup monomer variant in the present invention, any one of the Mmup monomer variants in the present invention, or any one of constructs in the present invention.

Preferably, the nucleotide sequence encoding the Mmup monomer variant has 70%, 75%, 80%, 85%, 90%, 91%, 92%, 93%, 94%, 95%, 96%, 97%, 98%, 99%, 99.5%, 99.9% homology with the sequence shown by SEQ ID NO: 2 or SEQ ID NO: 11.

In a specific embodiment of the present invention, the nucleotide sequence encoding the Mmup monomer variant is shown by SEQ ID NO: 11.

The fifth aspect of the present invention provides a vector comprising the nucleotide sequence encoding the Mmup monomer variant of the present invention, the porin of the present invention, or the construct of the present invention.

Preferably, the vector can provide a plasmid, virus or phage vector which has an origin of replication, a optional promoter used to express the nucleotide sequence, and a regulatory signal gene of the optional promoter. The vector can comprise one or more selectable marker genes, such as tetracycline resistance genes. The promoter and other expression-regulatory signals can be selected to be compatible with the host cell for which the expression vector is designed. The promoter is selected from T7, trc, lac, ara or λL promoter.

The Mmup monomer variants in the present invention can be prepared by chemical synthesis or recombinant manner, and are preferably prepared by recombinant manner.

Preferably, the vector comprises a promoter operably linked with the nucleotide sequence encoding any one of porin comprising at least one Mmup monomer variant in the present invention, any one of the Mmup monomer variants in the present invention, or any one of the constructs in the present invention.

Further preferably, the promoter is an inducible promoter or a constitutive promoter, wherein the inducible promoter is an acetamide inducible promoter.

Preferably, the nucleotide sequence encoding the porin comprising at least one Mmup monomer variant comprises at least one nucleotide sequence encoding the Mmup monomer variant.

Further preferably, the nucleotide sequence encoding the porin comprising at least one Mmup monomer variant also comprises at least one nucleotide sequence encoding the wild-type Mmup monomer.

Still further preferably, the nucleotide sequence encoding the Mmup monomer variant and the nucleotide sequence encoding the Mmup monomer variant, the nucleotide sequence encoding the Mmup monomer variant and the nucleotide sequence encoding the wild-type Mmup monomer, or, the nucleotide sequence encoding the wild-type Mmup monomer and the nucleotide sequence encoding the wild-type Mmup monomer are linked by sequences encoding amino acid linker.

The sixth aspect of the present invention provides a mutant bacterium expressing any one of the Mmup monomer variants in the present invention, any one of the constructs in the present invention, or any one of the porins comprising at least one Mmup monomer variant in the present invention, the bacterium comprises:
(a) The deletion of the wild-type Mmup monomer; and (b) any one of the vectors in the present invention.

Preferably, the bacterium comprises a vector of the promoter operably linked with the nucleotide sequence encoding the Mmup monomer variant, the construct comprising the Mmup monomer variant, or the porin comprising the Mmup monomer variant.

Further preferably, the Mmup monomer variant comprises a paralogue or homologue of the Mmup monomer variant.

Further preferably, the construct comprising the Mmup monomer variant comprises a paralogous or homologous construct or monomer of the Mmup monomer variant.

Further preferably, the porins comprising the Mmup monomer variant comprises a paralogous or homologous porin or monomer of the Mmup monomer variant.

Preferably, the bacterium can also comprise a vector of the promoter operably linked with the nucleotide sequence encoding the wild-type Mmup monomer, the construct comprising the wild-type Mmup monomer, or the porin comprising the wild-type Mmup monomer.

Further preferably, the wild-type Mmup monomer comprises a paralogous or homologous monomer of the wild-type Mmup monomer.

Further preferably, the construct comprising the wild-type Mmup monomer is a paralogous or homologous construct or monomer of the wild-type Mmup monomer.

Further preferably, the porin comprising the wild-type Mmup monomer is a paralogous or homologous porin or monomer of the wild-type Mmup monomer.

Preferably, the bacterium is *Mycobacterium mucinae.*

The seventh aspect of the present invention provides a method of producing Mmup porin, the method includes transforming any one of the bacteria in the present invention with any one of the vectors in the present invention, and inducing the bacteria to express the Mmup porin.

The eighth aspect of the present invention provides a method of preparing the Mmup monomer variant. The vector can be introduced into the suitable host cell by inserting the nucleotide sequence encoding the Mmup monomer variant into the vector, introducing the vector into the compatible bacterial host cell and culturing the host cell under conditions that allow the expression of the nucleotide to produce the Mmup monomer variant in the present invention.

The ninth aspect of the present invention provides a cell comprising the nucleotide sequence or the vector in the present invention.

Preferably, the cell can be *Escherichia coli,* etc. More preferably, the cell is a dam+ type strain (for example, a DH5α strain).

The tenth aspect of the present invention provides a method of characterizing the target polynucleotide, including:
(a) Contacting the target polynucleotide with any one of the porins comprising at least one Mmup monomer variant in the present invention, so that the target polynucleotide sequence passes through the porin comprising at least one Mmup monomer variant; and
(b) Obtaining one or more characteristics of the interaction between the nucleotide and the porin comprising at least one Mmup monomer variant when the target polynucleotide passes through the porin comprising at least one Mmup monomer variant to characterize the target polynucleotide.

Preferably, the steps (a) and (b) are repeated one or more times.

Preferably, the target polynucleotide in the step (a) can bind to the polynucleotide processing enzyme derived from the polynucleotide to control the translocation speed. Further preferably, the polynucleotide processing enzyme is a polypeptide capable of interacting with the polynucleotide and modifying at least one of the polynucleotide properties. Wherein, the polynucleotide processing enzyme can or can not have enzymatic activity, as long as the enzyme binds to the polynucleotide and controls the translocation speed of the polynucleotide in the pore. Wherein, the nucleic acid can bind to one or more polynucleotide processing enzymes.

Preferably, the polynucleotide processing enzyme is a nucleolytic enzyme. Further preferably, the polynucleotide processing enzyme comprises but is not limited to the nucleic acid binding protein, helicase, polymerase, exonuclease, telomerase, reverse transcriptase, translocase or topoisomerase.

In a specific embodiment of the present invention, the polynucleotide processing enzyme is a gyrase.

Preferably, the step (a) also includes the step of contacting the target polynucleotide with one or a combination of two or more of the nucleic acid binding protein, helicase, exonuclease, telomerase, topoisomerase, reverse transcriptase, translocase and/or the polymerases, which makes the translocation speed of the target polynucleotide sequence through the porin less than the translocation speed in the absence of the nucleic acid binding protein, helicase, exonuclease, telomerase, topoisomerase, reverse transcriptase, translocase, and/or polymerase.

Further preferably, the nucleic acid binding protein comprises but is not limited to one or a combination of two or more of the modified or wild eukaryotic single-stranded binding protein, bacterial single-stranded binding protein, archaeal single-stranded binding protein, viral single-stranded binding protein or double-stranded binding protein. The nucleic acid binding protein comprises but is not limited to, SSBEco from *Escherichia coli,* SSBBhe from *Bartonella henselae,* SSBCbu from *Coxiella burnetii,* SSBTma from *Thermathoga maritima,* SSBHpy from *Helicobacter pylori,* SSBDra from *Deinococcus radioticdurans,* SSBTaq from *Thermus aquaticus,* SSBMsm from *Mycobacterium smegmatis,* SSBSso from *Sulfolobus solfataricus,* SSBSso7D from *Sulfolobus solfataricus,* SSBMHsmt from *Homo sapiens,* SSBMle from *Mycobacterium leprae,* gp32T4 from *Bacteriohage T4,* gp32RB69 from *Bacteriophage RB69,* or gp2.5T7 from *Bacteriohage T7.*

Further preferably, the helicase can be any one of Hel308 family helicase and modified Hel308 family helicase, RecD helicase and its variants, TrwC helicase and its variants, Dda helicase and its variants, TraI Eco and its variants, XPD Mbu and its variants.

Further preferably, the polymerase comprises but is not limited to the modified or wild DNA polymerase, comprises but is not limited to Phi29 DNA polymerase, Tts DNA polymerase, M2 DNA polymerase, VENT DNA polymerase, T5 DNA polymerase, PRD1 DNA polymerase, Bst DNA polymerase or REPLI-gscDNA polymerase.

Further preferably, the exonuclease comprises but is not limited to the modified or wild exonuclease I from *E. coli,* the exonuclease III from *E. coli,* the exonuclease from *bacteriophageλ* or the RecJ from *Thermus thermophilus.* In a specific embodiment of the present invention, the step (a) comprises the step of contacting the target polynucleotide with the helicase, the helicase is EF8813, and the amino acid sequence of the helicase is showed by SEQ IDNO: 3, the nucleotide sequence of the helicase is shown by SEQ ID NO: 4. Preferably, the target polynucleotide can be contacted with one or more helicases. Further preferably, the target polynucleotide can be contacted with 2 to 20 helicases, or even more helicases. Wherein, the helicase that binds to the target polynucleotide can be identical or different. And the multiple helicases binding to the target polynucleotide are covalently linkd with each other.

Preferably, the one or more characteristics are selected from the source, length, size, molecular weight, identity, sequence, secondary structure, concentration of the target polynucleotide, or whether the target polynucleotide is modified.

In a specific embodiment of the present invention, the characteristic is a sequence.

Preferably, the one or more characteristic in the step (b) are performed by electrical measurement and/or optical measurement.

Further Preferably, the electrical and/or optical signals are generated by electrical measurement and/or optical measurement, and each nucleotide corresponds to a signal level, and then the electrical signals and/or optical signals are converted into the nucleotides sequence characteristics.

The electrical measurement in the present invention is selected from the current measurement, impedance measurement, field effect transistor (FET) measurement, tunnel measurement or wind tunnel measurement.

The electrical signal in the present invention is selected from the values of the current, voltage, tunneling, resistance, potential, conductivity or transverse electrical measurement.

In a specific embodiment of the present invention, the electrical signal is a current passing through the pore. That is, the current passes through the pore in a nucleotide-specificity manner, and if the characteristic current through the pore related to the nucleotides is detected, the existence of nucleotides can be proven. Otherwise, the nucleotide does not exist. However, the distinction between similar nucleotides or modified nucleotides is determined by the current amplitude.

Preferably, the conductance generated in the process of the polynucleotide characterization adopting the porin of the present invention is higher than that of adopting the pore formed by the wild-type Mmup monomer.

Preferably, the method also comprises the step of applying a potential difference across the porin contacted with the target polynucleotide. Wherein, the potential difference is sufficient to translocate the target polynucleotide from the channel of the porin.

Preferably, the target polynucleotide can be natural DNA, RNA, or modified DNA or RNA.

The target polynucleotide in the present invention is a macromolecule comprising one or more nucleotides.

The target polynucleotide in the present invention can be natural or artificially synthesized. Preferably, one or more nucleotides of the target polynucleotide can be modified, such as methylation, oxidation, damage, abasic, protein labeling, with tagging or a spacer linked in the middle of polynucleotide sequence. Preferably, the artificially synthesized nucleic acid is selected from peptide nucleic acid (PNA), glycerol nucleic acid (GNA), threose nucleic acid (TNA), locked nucleic acid (LNA), or other synthetic polymers with nucleoside side chains.

Preferably, the porin allows hydrated ions to flow from one side of the membrane to the other layer of the membrane under the driving of an applied electric potential. Wherein, the membrane can form a barrier of the flow of ions, nucleotides and nucleic acids. Further Preferably, the membrane is a double-layer membrane, and still further preferably a lipid bilayer membrane. The lipid bilayer membrane comprises but is not limited to one or a mixture of two or more of phospholipids, glycolipids, cholesterol, and mycolic acid.

Preferably, the porin channel is located between the first conductive liquid medium and the second conductive liquid medium, wherein at least one conductive liquid medium comprise the target polynucleotide, and the first conductive liquid medium and the second conductive liquid medium can be identical or different, as long as the purpose of analyzing one or more characteristics of the target polynucleotide can be achieved.

Preferably, the target polynucleotide is single-stranded, double-stranded, or at least a part of the target polynucleotide is double-stranded.

In a specific embodiment of the present invention, at least a part of the target polynucleotide is double-stranded. Wherein the double-stranded part constitutes a Y-adapter structure, the Y-adapter structure comprises the leader sequence that is preferentially screwed into the porin, and the 3'end of the leader sequence links with thiol, biotin or cholesterol, which aims to bind to a layer of the lipid bilayer membrane to point the target polynucleotide in the correct direction and have a pulling effect.

In a specific embodiment of the present invention, the 3'end of the leader sequence links with cholesterol to bind to a layer of the lipid bilayer membrane.

Adjusting the voltage, salt concentration, buffer, additives, or temperature in the process of characterizing the target polynucleotide can control the differentiation degree of different nucleotides by adopting the porin of the present invention in the process of characterizing the target polynucleotide. Wherein, the additive is selected from DTT, urea or betaine.

Preferably, the voltage range is from -250mV to +250mV. Further preferably, the voltage is selected from -250mV, -210mV, -180mV, -140mV, -110mV, -90mV, -70mV, -40mV, 0mV, +40mV, +70mV, +90mV, +110mV, +140mV, +180mV, +210mV, +250mV

In a specific embodiment of the present invention, the voltage is from -180mV to +180mV.

In a specific embodiment of the present invention, the method includes: inserting the porin into the membrane, and contacting the target polynucleotide with the porin, nucleic acid binding protein, polymerase, exonuclease, telomerase, topoisomerase, reverse transcriptase, translocase and/or helicase, applying a potential difference across the porin contacted with the target polynucleotide, so that the target polynucleotide sequence passes through the porin; and

Obtaining the current characteristics of the interaction between the nucleotide and the porin when the target polynucleotide passes through the porin to identify whether the polynucleotide is present, what kind of nucleotide it is, or whether the polynucleotide is modified.

Preferably, the method of inserting the porin into the membrane can be any method known in the field that can achieve the purpose of characterizing the polynucleotide. Further preferably, the porin can be suspended in the solution containing the lipid bilayer in a purified form, so that the porin can diffuse into the lipid bilayer and is inserted into the lipid bilayer by binding to the lipid bilayer and being assembled into a functional state.

The eleventh aspect of the present invention provides an application of any one of the porins comprising at least one Mmup monomer variant in the the present invention, any one of the Mmup monomer variants in the present invention, any one of the constructs in the present invention, the nucleotide sequences in the present invention, any one of the vectors in the present invention, or any one of the mutant bacteria of the present invention in the process of characterizing the target polynucleotide.

The twelfth aspect of the present invention provides a kit for characterizing the target polynucleotide. The kit comprises any one of the porins in the present invention comprising at least one Mmup monomer variant, any one of the Mmup monomer variants in the present invention, any one of the constructs in the present invention, the nucleotide sequences in the present invention, any one of the vectors in the present invention, or any one of the mutant bacteria in the present invention.

Preferably, the Mmup monomer variant, the construct, the nucleotide sequence, the vector, the cell or the porin can be multiple.

Preferably, the kit also comprises one or a combination of two or more of one or more nucleic acid binding proteins, helicases, exonucleases, telomerases, topoisomerases, reverse transcriptases, translocases and/or polymerases.

Preferably, the kit also comprises the lipid bilayer chip, and the porin across the lipid bilayer.

Preferably, the kit comprises one or more lipid bilayers, and each lipid bilayer comprises one or more porins.

Preferably, the kit also comprises the reagents or devices for performing to characterize the target polynucleotide. Further preferably, the reagent comprises the buffers and tools required for PCR amplification.

The thirteenth aspect of the present invention provides a device for characterizing the target polynucleotide, the device comprises any one of the porins comprising at least one Mmup monomer variant in the present invention, any one of the Mmup monomer variants in the present invention, any one of the constructs in the present invention, the nucleotide sequences in the present invention, any one of the vectors in the present invention, or any one of the mutant bacteria in the present invention.

Preferably, the device also comprises one or a combination of two or more of one or more nucleic acid binding proteins, helicases, exonucleases, telomerases, topoisomerases, reverse transcriptases, translocases and/or polymerases.

Preferably, the device also comprises the sensor that supports the porin and can transmit the signal that the porin interacts with the polynucleotide, at least one memorizer for storing the target polynucleotide, and the solution required for performing the characterizing process .

Preferably, the device also comprises a patch clamp amplifier and/or a data acquisition device.

The fourteenth aspect of the present invention provides a sensor for characterizing target polynucleotide. The sensor comprises any one of the porins comprising at least one Mmup monomer variant in the present invention, any one of the Mmup monomer variants in the present invention, any one of the constructs in the present invention, the nucleotide sequences in the present invention, any one of the vectors in the present invention, or any one of the mutant bacteria in the present invention.

The "non-natural amino acid" in the present invention is a compound containing amino and carboxyl groups that is not naturally found in proteins. Preferably, the non-natural amino acid is any non-natural amino acid known in the field. Further preferably, the non-natural amino acid comprises but is not limited to N-ethyl aspartyl, hydroxylysine, 3-hydroxyproline, 2-aminobutyric acid, β-alanine, β-amino Propionic acid, 2-aminoadipate, 3-aminoadipate, 4-aminobutyric acid, 6-aminocaproic acid, 2-amino heptanoic acid, allo-isoleucine, isochainlysine, 4-Hydroxyproline, allo-hydroxylysine, 2-aminoisobutyric acid, N-methylglycine, N-methy lisoleucine, 3-aminoisobutyric acid, 6-N-methyllysine, 2,4-Diaminobutyric acid, N-methylvaline, ornithine, norleucine, norvaline, desmosine, 2,2'-diaminopimelic acid, 2,3-Diamino propionic acid, N-ethyl glycine or 2-amino heptanedioic, etc.

The "modified... amino acid" in the present invention is an amino acid of which the side chain is chemically modified. For example, the post-translationally modified amino acids, or the amino acids with side chains comprising novel functional groups (such as sulfhydryl, amino, or carboxyl), or side chains comprising signal-generating moieties (such as fluorescent groups or radiolabels).

The "nucleotide" in the present invention comprises but is not limited to: adenosine monophosphate (AMP), guanosine monophosphate (GMP), thymidine monophosphate (TMP), uridine monophosphate (UMP), cytosine nucleoside monophosphate (CMP), cyclic adenosine monophosphate (cAMP), cyclic guanosinemonophosphate (cGMP), deoxyadenosine monophosphate (dAMP), deoxyguanosine monophosphate(dGMP), deoxythymidine monophosphate (dTMP), deoxyuridine monophosphate (dUMP) anddeoxycytidine monophosphate (dCMP). Preferably, the nucleotide is selected from AMP, TMP, GMP, CMP, UMP, dAMP, dTMP, dGMP or dCMP.

The "and/or" in the present invention comprises the alternatively listed items and the combination of any number of items.

The "comprise", "contain" and "include" in the present invention is an open-ended description, comprising the specified components or steps described, and other specified components or steps that can not have an effect substantially .

The "approximately" and "about" in the present invention is used to indicate the value and the standard deviation allowed by the device or method for determine the value.

The "homology", "homologue" and "homologous" in the present invention refers to, in terms of utilizing protein sequences or nucleotide sequences, the technicists in the field can adjust the sequence based on actual work needs to make the used sequence have (comprising but not limited to)1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99 %, 99.1%, 99.2%, 99.3%, 99.4%, 99.5%, 99.6%, 99.7%, 99.8%, 99.9% sequence identity compared with the sequence obtained by the current technology.

The "Mmup monomer variant" in the present invention refers to the Mmup monomer variant which has at least or at most 70, 75, 80, 85, 90, 91, 92, 93, 94, 95, 96, 97, 98, 99, 99.5 or 99.9% or more, or a value from any range in between, but less than 100% identity with the wild-type Mmup monomer, and remains the ability of forming the channel when binding to one or more other Mmup monomer variants or wild-type Mmup monomers. Optionally, the Mmup monomer variant is further determined to comprise the mutation in the part of the sequence that promotes the formation of the constriction and/or loops region of the fully formed channel-forming porin. The Mmup monomer variant can be, for example, a recombinant protein. The Mmup monomer variant can comprise any mutation in the present invention.

The "paralogous or homologous porin of the Mmup monomer variant " in the present invention refers to the paralogous or homologous porin of the Mmup monomer variant which has at least or at most 70, 75, 80, 85, 90, 91, 92, 93, 94, 95, 96, 97, 98, 99, 99.5, or 99.9% or more, or a value from any range in between, but less than 100% identity with the paralogous or homologous porin of the wild-type Mmup monomer and retains the ability of forming the channel. Optionally, the paralogous or homologous porin of the Mmup monomer variant is further determined to comprise the mutation in the part of the sequence, that promotes the formation of the constriction and/or loops region of the fully formed channel forming porin. The paralogous or homologous porin of the Mmup monomer variant can be, for example, a recombinant protein. Any paralogous or homologous porin of the Mmup monomer variant can optionally be used in any embodiment in the present invention.

The "paralogous or homologous construct of the Mmup monomer variant" in the present invention refers to the paralogous or homologous construct of the Mmup monomer variant which has at least or at most 70, 75, 80, 85, 90, 91, 92, 93, 94, 95, 96, 97, 98, 99, 99.5, or 99.9% or more, or a value from any range in between, but less than 100% identity with the paralogous or homologous construct of the wide-type Mmup monomer variant and retains the ability of forming the channel. Optionally, the paralogous or homologous construct of the Mmup monomer variant is further determined to comprise the mutation in the part of the sequence, that promotes the formation of the constriction and/or loops region of the fully formed channel-forming porin. The paralogous or homologous construct of the Mmup monomer variant can be, for example, a recombinant protein. Any paralogous or homologous construct of the Mmup monomer variant can optionally be used in any embodiment in the present invention.

### Instruction with figures

Herein after, the embodiments of the present invention are described in detail with reference to the pictures, wherein:
Figure 1: Purification results of Mmup-(D91K/A99K) monomer variant protein size exclusion chromatography. Lanes 1 to 6 show the SDS-PAGE electrophoresis detection results of different components separated by molecular sieves.

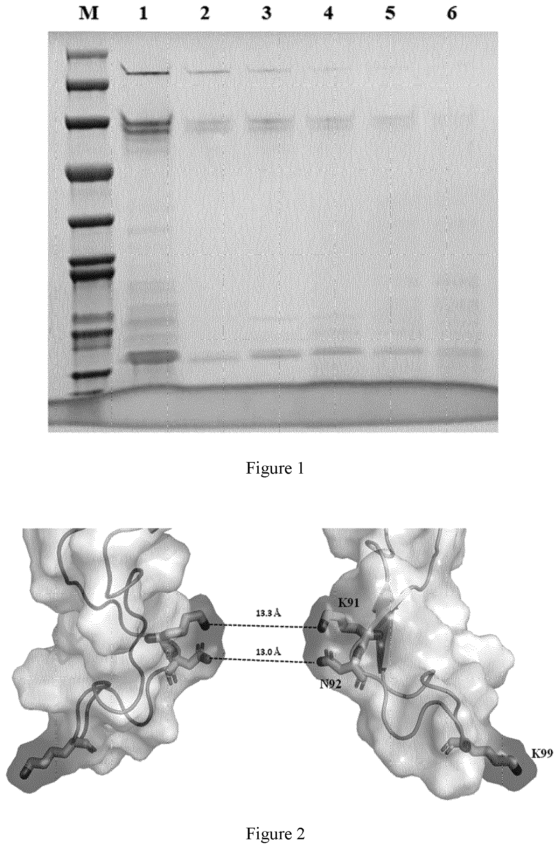

Figure 2: The stick model of the nanoporin (D91K/A99K) comprising Mmup monomer variants. The figure mainly shows the amino acid distribution characteristics of the constriction and loops region of the pore channel, especially the key amino acid residues distribution of the constriction and the loops region, the lysine at 91st site and the asparagine at 92nd site of amino acid residues pointing to the center of the pore channel, the diameter of the pore channel is approximately 13Å, lysine at 99th site is involved in the correct assembly of the channel complex wherein homology modeling is constructed by SWISS MODEL, the template pdb is 1uun.
Figure 3: The stick model of the nanopore channel comprising the wild-type Mmup monomers. The figure mainly shows the amino acid distribution characteristics of the constriction and the loops region of the pore channel, especially the key amino acid residues of the constriction and loops region, mainly aspartic at 91st site, asparagine at 92nd site, valine at 93rd site and serine at 94th site, the diameters of the constriction formed by D91 and N92 are respectively 15.5Å and 13.5Å, wherein the homology modeling is constructed by SWISS MODEL, the template pdb is 1uun.
Figure 4: The cartoon diagram of the nanopore comprising the wild-type Mmup monomer based on homology modeling. wherein, the region 1 corresponds to the cap forming region, the region 2 corresponds to the barrel forming region, and the region 3 corresponds to the constriction and the loops region.

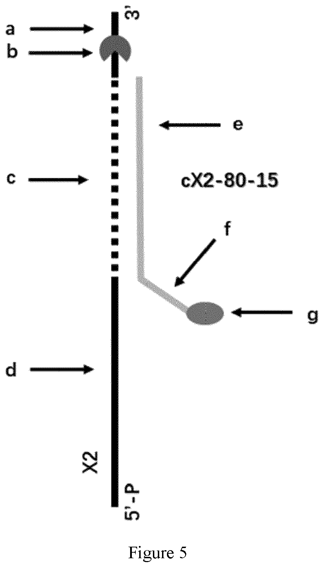

Figure 5: The structure diagram of the DNA construct X2&cX2-80-15 to be tested, wherein segment a corresponds to SEQ ID NO: 7, and segment b corresponds to helicase EF8813-1 (A variant protein comprising N-terminal histidine tag and fused with TOPV-HI domain, SEQ ID NO: 3-4), the helicase can bind to the segment a, segment c corresponds to SEQ ID NO: 6, segment d corresponds to SEQ ID NO: 5, segment e corresponds to SEQ ID NO: 8, the 45 bases at the 5'end of cX2-80-15 are complementary to the test strand segment c for pairing, and the 3'end of cX2-80-15 contains 40 thymines and the 3'cholesterol TEG tag corresponding to g , segment f corresponds to SEQID NO: 9.

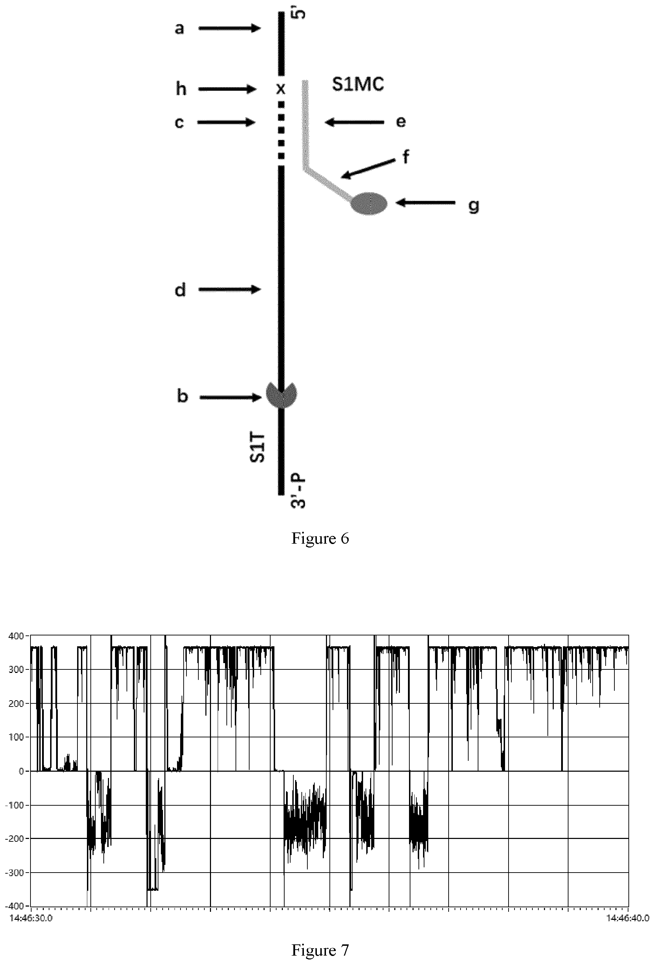

Figure 6: The structure diagram of the DNA construct S1T&S1MC to be tested, wherein segment a corresponds to SEQID NO: 10, and segment b corresponds to helicase EF8813-1 (A variant protein comprising N-terminal histidine tag and fused with TOPV-HI domain, SEQ ID NO: 3-4) , the helicase can bind to the segment a, segment h refers to the dspacer that only retains the phosphate backbone, labeled x, segment c corresponds to SEQ ID NO: 12, segment d corresponds to SEQ ID NO: 13, segment e corresponds to SEQ ID NO: 14, the 45 bases at the 5'end of S1MC are complementary to the test strand segment c for pairing, and the 3'end of S1MC contains 20 thymines and the 3'cholesterol TEG tag corresponding to g, segment f corresponds to SEQ ID NO: 15.
Figure 7: Single-channel behavior characteristics of the nanopore channel comprising the wild-type Mmup monomer at a voltage of ±180 mV, wherein the y-axis coordinate = current (pA), and the x-axis coordinate= time (s).
Figure 8: Open-pore current and gating characteristics of the nanoporin comprising the Mmup monomer variants (D91K/A99K) at a voltage of +180mV, 0mV and -180mV, wherein y-axis coordinate = current (pA), x-axis coordinates = time (s).
Figure 9: Signal characteristics of the nanoporin containing the Mmup monomer variants (D91K/A99K) at a voltages of +180mV and 0mV when the nuclear acid passes through the nanopore, wherein the y-axis coordinate = current (pA), and the x-axis coordinate = time (s).
Figure 10: The part of exemplary current trace when the helicase (EF8813-1) controls the translocation of the DNA construct X2 through the nanoporin comprising the Mmup monomer variant (D91K/A99K), wherein the y axis coordinates of the two traces (left -20 to 180, right 10 to 80) = current (pA), x axis coordinates (left 15:04:25.5 to 15:04:37.5, right 15:04:34.1 to 15:04:37.1) = Time (s), the right figure is the amplified result of the current trace showed by the dotted line in the left figure.
Figure 11: The whole process current change results of the current trace when the helicase (EF8813-1) controls the translocation of the DNA construct X2 through the nanoporin comprising the Mmup monomer variant (D91K/A99K), wherein y axis coordinate = current (pA), x axis coordinate = time (s).
Figure 12: The part of exemplary current trace when the helicase (EF8813-1) controls the translocation of the DNA construct SIT through the nanoporin comprising the Mmup monomer variant (D91K/A99K), wherein the y-axis coordinate of the two traces =current (pA), x-axis coordinate=time (s), the right figure is the amplified result of the current trace shown by the dotted line in the left figure, and the current maximum indicated by the arrow shows the characteristic peak of dspacer.
Figure 13: The whole process current change results of the current trace when the helicase (EF8813-1) controls the translocation of the DNA construct SIT through the nanoporin comprising the Mmup monomer variant (D91K/A99K), wherein y axis coordinate = current (pA), x axis coordinate = time (s).

### Concrete Embodiments

The technical solutions in the embodiments of the present invention are described clearly and completely with the reference to the figures in the embodiments of the present invention. Obviously, the described embodiments are only a part of the embodiments of the present invention but not all. Based on the embodiments of the present invention, all other embodiments obtained by those of the ordinary skill in the field without creative work belongs to the protection scope of the present invention.

### Example 1 Preparation of the Mmup monomer variant

### 1. Plasmid construction

The protein sequence of the Mmup monomer variant was optimized by the corresponding amino acid codons, and appropriate restriction endonuclease sites were added at both ends of the gene. Specificlly, the NcoI site ccatgg was added at the 5'end , and the xhoI site ctcgag was added at the 3'end, then performed the gene synthesis and the synthesized gene was cloned into the expression vector pET24b.

### 2. Site-directed mutagenesis of the target gene, preparation of the nucleotide sequence of the Mmup monomer variant

Induced the mutant gene (PCR reaction), taked the plasmid to be mutated as the template, and used the designed primers and KOD plus high-fidelity enzyme to perform PCR amplification reaction to induce the target gene mutation.

Concrete steps are as follows:
1. Designed the point mutation primers, prepared the template plasmid DNA (comprising the plasmid DNA of SEQ IDNO: 2), and performed 50 µL PCR reaction system amplification. The DH5α strain was used as the host bacteria. In end+ strains, the number of clones is often low, but which has no effect in the mutation efficiency. Wherein, extracted the template plasmid DNA using QIGEN plasmid purification kit.
The point mutation primers:
SEQ ID NO: 16 
GACCCCGAACATTTTACTGAAAAATGTGAGCCCGGCGACCCTGAAAGGTGTTGGTTG
GGGTGGC
SEQ ID NO: 17 
GCCACCCCAACCAACACCTTTCAGGGTCGCCGGGCTCACATTTTTCAGTAAAATGTTC
GGGGTC
50µE PCR reaction system:

| | |
|---|---|
| 10×KOD plus Buffer | 5µL |
| Template plasmid DNA 60ng | 2µL |
| Forward primer (20pmol/µL) | 2µL |
| Reverse primer (20pmol/µL) | 2µL |
| dNTP mixture (each 2.5mM) | 2µL |
| MgSO₄ | 2.5µE |
| KOD plus enzyme | 1µL |
| ddH₂O | 33.5µL |

PCR amplification reaction
Cycles, temperature and reaction time:

| | | |
|---|---|---|
| 1 cycle | 95°C | 5min |
| 18 cycles | 95°C | 30s |
| | 72°C | 30s 6min |
| | 55°C | 30min |
| 1 cycle | 72°C | 10min |

The PCR amplification reaction was finished, and the nucleotide sequence of the Mmup monomer variant (SEQ ID NO: 11) was obtained , and then it was placed in an ice bath for 5 minutes and then placed at room temperature (avoid repeated freezing and thawing).

### 2. Template digestion, extraction of the Mmup monomer variant gene

After the PCR reaction was finished, the methylated plasmid was digested with DpnI enzyme to select mutant plasmid DNA (the plasmid comprising SEQ ID NO: 11). Prepared PCR reaction products. The specific steps were as follows: added 1 µL (10U/µL) DpnI enzyme and incubated for 2 hours at 37°C. (When the amount of the plasmid DNA is too much, DpnI enzyme may react incompletely with the sample. If the mutation rate is low, the reaction time can be prolonged or the amount of DpnI Enzyme can be increased appropriately)

### 3. Transformation, acquistion of the strain comprising the Mmup monomer variant gene

After the reaction, the gaps in the plasmid DNA were generated. Therefore, DH5α was selected when transforming the plasmid DNA into *E. coli.* The specific steps were as follows: added 4µL mutant plasmid DNA sample to 50µL DH5α competent cells, then placed it on ice for 30min, heated shock at 42°C for 90s, then immediately ice bath for 2min, added 500µL SOC medium and cultured at 37°C for 1 hour, and finally taked 100 µL bacterial solution to coat the resistance screening plate.

### 4. Sequencing verification

Picked four transformants for culturing and sequencing, and selected the positive transformants with correct mutation to extract plasmids and saved them for next step.

### 3. Preparation of the Mmup monomer variant

The Mmup monomer variant plasmid verified by sequencing correctly was transformed into BL21 (DE3) for culture. Then the protein was purified, and the reagent formula for protein purification is shown in Table 1.

Pipetted 20µL of BL21(DE3) glycerol bacteria containing the Mmup monomer variant plasmid into 20mL (1:1000) fresh LB medium with a final concentration of 50mg/mL kanamycin, 37°C, 200rpm shaked overnight to activate; The next day expanded to cultivate in 1% inoculation amount into 2L LB medium with a final concentration of 50mg/mL kanamycin. After culturing at 37°C, 220 rpm to OD600 = 0.6-0.8, quickly cooled in an ice bath. Then added IPTG to the culture system to a final concentration of 1 mM, induced the expression overnight at 15° C and 220 rpm. The next day, collected the bacteria by centrifugation at 6000 rpm and 4°C for 15 minutes. Resuspended the bacteria in a ratio of bacteria: lysis buffer = 1:10 (m/v), and then added mixed protease inhibitors and TritonX-100 at a final concentration of 2%, high-pressure crushed until the bacteria liquid became clear.

At room temperature, stirred and solubilized for 1 to 2 hours, centrifuged at 14000 rpm, 4°C for 30 min, and collected the supernatant. The supernatant was filtered with a 0.45µm filter membrane and purified with an anion exchange column. The ion column was pre-equilibrated with Buffer B. The supernatant was passed through the column at a flow rate of 5 mL/min, and the penetrating solution was collected. Then used Buffer B to elute the impurity protein, and finally used Buffer C: 0-1M salt concentration linear gradient to elute to collect the eluted fractions. The collected penetrating samples were added with ammonium sulfate with a final concentration of 40%, and precipitated in an ice bath for 2 hours, and then centrifuged at 14000 rpm and 4°C for 30 minutes to collect floating precipitates. The floating precipitates were re-dissolved by adding a certain volume of molecular sieve buffer with 0.5% C8E4 (tetraethylene glycol monooctyl ether) detergent, and incubated overnight at 4°C.

Next day, incubation sample overnight was centrifuged at 47000g, 4°C for 30 min, and the supernatant was collected. The supernatant was performed to the last step of size exclusion chromatography purification, and the collected target component was the Mmup monomer variant. The results of size exclusion chromatography are shown in Figure 1.

**Table 1 Reagent formulations for protein purification**

| Reagent items | Components |
|---|---|
| Lysis Buffer | 50mM Tris-HCl 8.0 |
| | 150mM NaCl |
| | 10% Glycerol |
| Buffer B | 25mM HEPES-Na 7.5 |
| | 0.5% OPOE |
| Buffer C | 25mM HEPES-Na 7.5 |
| | 1M NaCl |
| | 0.5% OPOE |

### Example 2 Preparation of the porin

Pipetted 20µL of BL21(DE3) in glycerol containing the Mmup monomer variant plasmid into 20mL (1:1000) fresh LB medium with a final concentration of 50mg/mL kanamycin, 37°C, 200rpm shaked overnight to activate; The next day expanded to cultivate in 1% inoculation amount into 2L LB medium with a final concentration of 50mg/mL kanamycin. After culturing at 37°C, 220 rpm to OD600 = 0.6-0.8, quickly cooled in an ice bath. Then added IPTG to the culture system to a final concentration of 1 mM, induced the expression overnight at 15° C and 220 rpm. The next day, collected the bacteria by centrifugation at 6000 rpm and 4°C for 15 minutes. Resuspended the bacteria in a ratio of bacteria: lysis buffer = 1:10 (m/v), and then added mixed protease inhibitors and TritonX-100 at a final concentration of 2%. High-pressure crushed until the bacteria liquid became clear.

At room temperature, stirred and solubilized for 1 to 2 hours, centrifuged at 14000 rpm, 4°C for 30 min, and collected the supernatant. The supernatant was filtered with a 0.45µm filter membrane and purified with an anion exchange column. The ion column was pre-equilibrated with Buffer B. The supernatant was passed through the column at a flow rate of 5 mL/min, and the penetrating solution was collected. Then used Buffer B to elute the impurity protein, and finally used Buffer C: 0-1M salt concentration linear gradient to elute to collect the eluted fractions. The collected penetrating samples were added with ammonium sulfate with a final concentration of 40%, and precipitated in an ice bath for 2 hours, and then centrifuged at 14000 rpm and 4°C for 30 minutes to collect floating precipitates. The floating precipitates were re-dissolved by adding a certain volume of molecular sieve buffer with 0.5% C8E4 detergent, and incubated overnight at 4°C.

Next day, incubation sample overnight was centrifuged at 47000g, 4°C for 30 min, and the supernatant was collected. The supernatant was performed to the last step of size exclusion chromatography purification, and the collected target component was the Mmup monomer variant.

### Example 3 Sequencing application of the porin

In a buffer (400 mM KCl, 10 mM HEPES pH 8.0, 50 mM MgC12), a single nanoporin was inserted into the phospholipid bilayer, and the values of electrical measurement were obtained from the single nanoporin.

Specific steps are as follows:
After inserting the single porin (the Mmup monomer variant porin, stick model shown in Figure 2) of the amino acid with D91K/A99K mutation of SEQ ID NO: 1 into the phospholipid bilayer, the buffer(400mM KCl, 10 mM HEPES pH 8.0, 50 mM MgCl₂) flowed through the system to remove any excess Mmup monomer variant porin. Added DNA construct X2&cX2-80-15 or S1T&S1MC (1∼2nM final concentration) into the Mmup monomer variant porin experimental system, mixed uniformly, maked the buffer (400mM KCl, 10mM HEPES pH 8.0, 50mM MgCl₂ ) flow through the system to remove any excess DNA construct X2&cX2-80-15 or S1T&S1MC. Then added the premix of helicase (EF8813-1, 15nM final concentration) and fuel (ATP 3mM final concentration) into the porin experimental system of the single Mmup monomer variant, and monitored sequencing situation of the Mmup monomer variant porin at a voltage of +180mV.

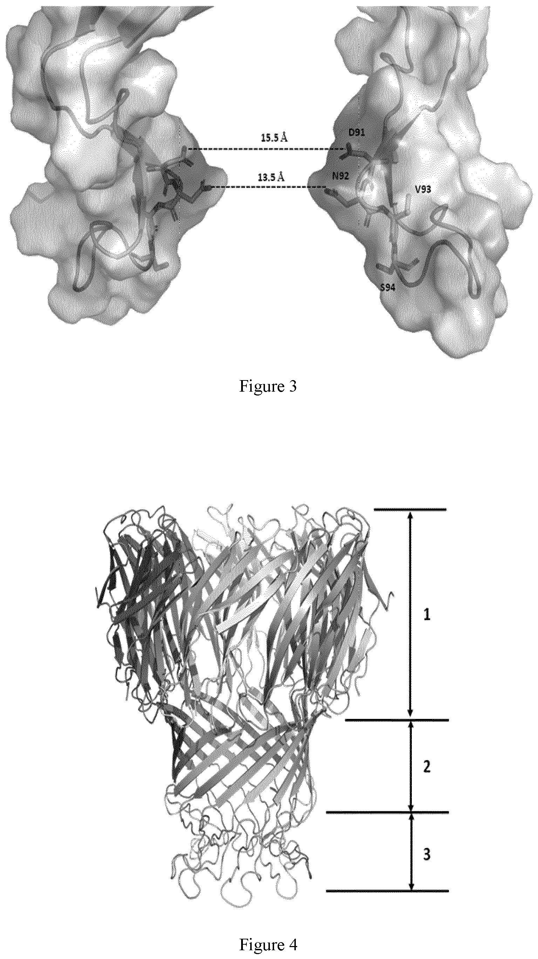

The control group is the same as the above steps, only replace the porin of the Mmup monomer variant with the nanopore of the wild-type Mmup monomer (the stick model and the three-dimensional structure are shown in Figures 3 and 4). wherein, the stick model of the nanopore of the wild-type Mmup monomer shows the amino acid distribution characteristics of the constriction and loops region of the pore channel, especially the key amino acid residues of the constriction and loops region, mainly aspartic at 91st site, asparagine at 92nd site, valine at 93rd site and serine at 94th site, the diameters of the constriction formed by D91 and N92 respectively are 15.5Å and 13.5Å. Compared with the nanopore of wild-type Mmup monomer, the stick model of the nanoporin (the stick model is shown in Figure 2) comprising the Mmup monomer variant shows the amino acid distribution characteristics of the constriction and loops region of the pore channel after mutation, especially the distribution of the key amino acid residues of the constriction and loops region, the amino acid residues of the lysine at 91st site and the asparagine at 92nd site pointing to the center of the pore channel, the diameter of the pore channel is approximately 13Å, the lysine at 99th site are involved in the correct assembly of the channel complex.

Wherein, the specific sequence of X2&cX2-80-15(the specific structure is shown in Figure 5) is as follows:
X2:
   TGGTTTTTGTTTGTTTTTAGAATTTTTTTACACTACCACTGCTAGCATTTTTCA (SEQ ID NO: 5) 
TTTCTCACTATCCCGTTCTCATTGGTGCACCATCTTTTTTTGGTT(SEQ ID NO: 6)
TTTTTGCAGCAGCAT (SEQ ID NO: 7)
cX2-80-15:
   AACCAAAAAAAGATGGTGCACCAATGAGAACGGGATAGTGAGAAA (SEQ ID NO: 8)
   TTTTTTTTTTTTTTTTTTTTTTTTTTTTTTTTTTTTTTTTTT (SEQ ID NO: 9)
wherein, the specific sequence of S1T&S1MC (the specific structure is shown in Figure 6) is as follows:
   S1T:
      TTTTTTTTTTTTTTCCTTCC (SEQ ID NO: 10)
   X (segment h)
      TTCTTTTCCCGTCCGCTCGT (SEQ ID NO: 12) 
TCGCGCCTGTCTGCTTGTTTTTTTTTTTTTCTTTTTTTTTTTTCTCACTATCGCATTCT

CATGCAGGTCGGTGGTCGCAGTA (SEQ ID NO: 13)
   S1MC:
      ACGAGCGGACGGGAAAAGAA (SEQ ID NO: 14)
      TTTTTTTTTTTTTTTTTTTT (SEQ ID NO: 15)

The test results are shown in Figure 7-13, and Figure 7 shows the single channel behavior characteristics of the nanopore channel comprising wild-type Mmup monomer at a voltage of ±180mV. The fully open current of the nanopore channel comprising the wild-type Mmup monomer in the test system is approximately 380pA under the +180mV condition, with obvious gating and strong residual nucleic acid through-pore signal; under the -180mV condition, the fully open current is close to -350pA, the gating is stronger, and the disturbance to the membrane is very violent. Obviously, the nanopore channel comprising the wild-type Mmup monomer cannot meet the requirements for sequencing nanoporins and cannot complete the sequencing purpose.

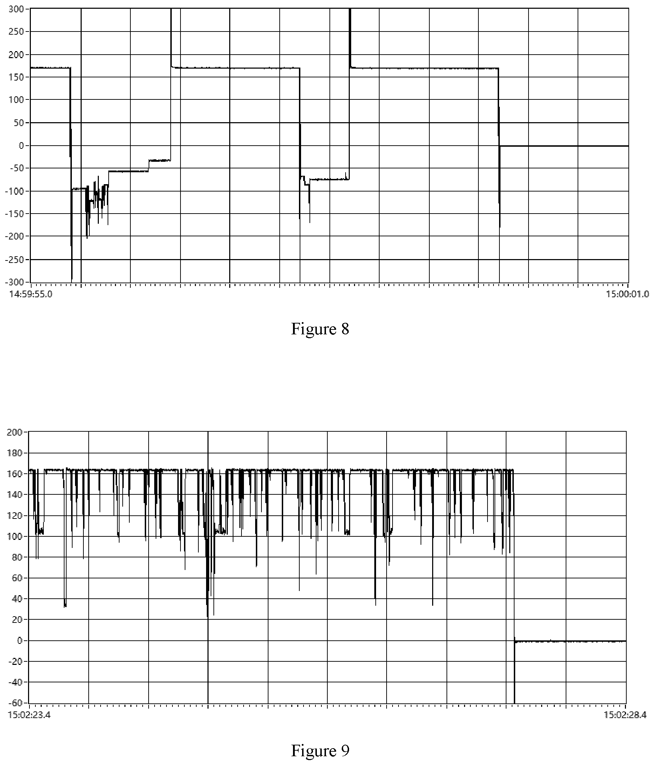

Figure 8 shows the open-pore current and its gating characteristics of the nanoporin comprising the Mmup monomer variant (D91K/A99K) at the voltage of +180mV, 0mV and -180mV, the positive gating disappears, and the porin can maintain a stable open state under applied voltage. At the condition of 180mv and 400mm KCl salt concentration, an open-pore current of about 160pA can be generated. Figure 9 shows the signal of the nucleic acid through the nanopore comprising the Mmup monomer variants (D91K/A99K) under the voltages of +180 mV and 0 mV

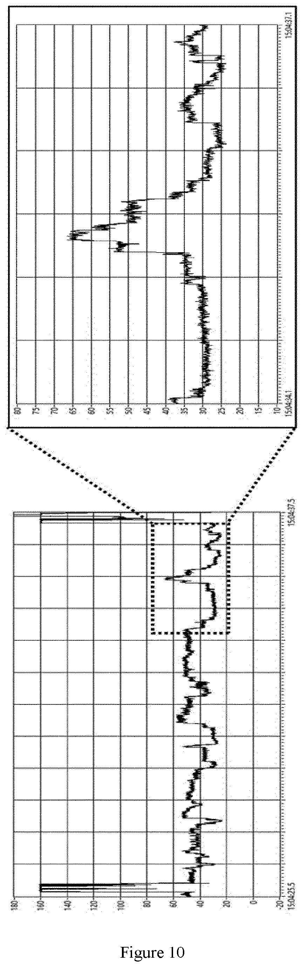

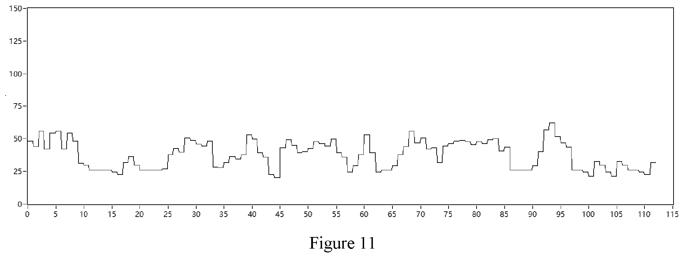

The part of exemplary current trace when the helicase (EF8813-1) controls the translocation of the DNA construct X2 through the nanoporin comprising the Mmup monomer variant (D91K/A99K). The right figure is the amplified result of the current trace with time 15:04:34.1 to 15:04:37.1 showed by the dotted line in the left figure(15:04:34.1 to 15:04:37.1) (see Figure 10). The whole process current change results of the current trace when the helicase (EF8813-1) controls the translocation of the DNA construct X2 through the nanoporin comprising the Mmup monomer variant (D91K/A99K) are shown in Figure 11.

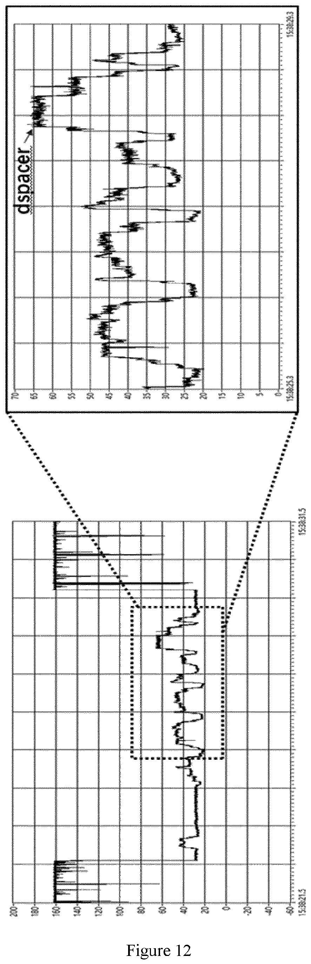

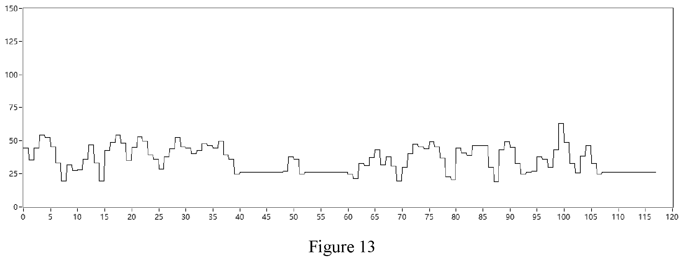

The part of exemplary current trace when the helicase (EF8813-1) controls the translocation of the DNA construct SIT through the nanoporin comprising the Mmup monomer variant (D91K/A99K), the right figure is the amplified result of the current trace shown by the dotted line in the left picture, wherein the current maximum indicated by the arrow shows the characteristic peak of dspacer (see Figure 12). The whole process current change results of the current trace when the helicase (EF8813-1) controls the translocation of the DNA construct SIT through the nanoporin comprising the Mmup monomer variant (D91K/A99K) are shown in Figure 13.

The preferred embodiments of the present invention are described in detail as above. However, the present invention is not limited to the specific details in the above-mentioned embodiments. Within the scope of the technical conception in the present invention, various simple modifications can be applied to the technical solution of the present invention. These simple modifications all belong to the protection scope of the present invention.

In addition, it should be noted that the various specific technical features described in the above specific embodiments can be combined in any suitable way without contradiction. In order to avoid unnecessary repetition, in the present invention, various possible combinations will not be explained additionally.

## Claims

1. A Mmup monomer variant, **characterized in that** the Mmup monomer variant comprises an amino acid sequence with any one or more amino acid mutations at 91st to 99th sites of SEQ ID NO: 1.

2. The Mmup monomer variant according to claim 1, **characterized in that** the Mmup monomer variant comprises a mutation of aspartic (D) at 91st site and/or alanine (A) at 99th site.

3. The Mmup monomer variant according to claim 1 or 2, **characterized in that** the Mmup monomer variant comprises at least one of the following mutations:
Aspartic (D) at 91st site is mutated to: proline (P), tryptophan (W), arginine (R), glutamine (Q), lysine (K), phenylalanine (F), serine (S ), asparagine (N), cysteine (C), isoleucine (I), leucine (L) or valine(V), or non-natural amino acid; or,
Alanine (A) at 99th site is mutated to: proline (P), phenylalanine (F), isoleucine (I), leucine (L), valine (V), lysine(K) or arginine (R), or, non-natural amino acid.

4. The Mmup monomer variant according to any one of claims 1-3, **characterized in that** the Mmup monomer variant comprises mutations of D91K and/or A99K.

5. The Mmup monomer variant according to any one of claims 1-4, **characterized in that** the Mmup monomer variant comprises mutations of D91K and A99K.

6. The Mmup monomer variant according to any one of claims 1-5, **characterized in that** the Mmup monomer variant also comprises an amino acid sequence with any one or more amino acid mutations at 80th to 90th sites and/or 100th to 120th sites of SEQ ID NO: 1.

7. The Mmup monomer variant according to any one of claims 1-6, **characterized in that** the Mmup monomer variant also comprises an amino acid sequence with any one or more amino acid mutations at 1^{st} to 79^{th} sites and/or 121^{st} to 186^{th} sites of SEQ ID NO: 1.

8. The Mmup monomer variant according to any one of claims 1-7, **characterized in that** the Mmup monomer variant also comprises one or a combination of two or more of a mutation of leucine (L) at 89^{th} site, a mutation of asparagine (N) at 110^{th} site, a mutation of aspartic (D) at 120^{th} site, a mutation of asparagine (N) at 136^{th} site or a mutation of serine (S)at 141^{st} site.

9. The Mmup monomer variant according to any one of claims 1-8, **characterized in that** the Mmup monomer variant comprises at least one of the following mutations:
Leucine (L) at 89^{th} site is mutated to: asparagine (N), alanine (A), glutamine (Q), glycine (G), serine (S), threonine(T), lysine (K) or proline (P), or, non-natural amino acid; or,
Asparagine (N) at 110^{th} site is mutated to: proline (P), alanine (A), isoleucine (I) or leucine (L), or non-natural amino acid; or,
Aspartic (D) at 120^{th} site is mutated to: arginine ^{®}, lysine (K), asparagine (N), glutamine (Q), alanine (A), serine (S), glycine (G) or threonine (T), or non-natural amino acid; or,
Asparagine (N) at 136^{th} site is mutated to: arginine ^{®}, lysine (K), glutamine (Q), alanine (A), serine (S) or threonine(T), or non-natural amino acid; or,
Serine (S) at 141^{st} site is mutated to: arginine ^{®}, lysine (K), glutamine (Q), alanine (A), serine (S) or threonine (T), or non-natural amino acid.

10. A construct comprising at least one Mmup monomer variant of any one of claims 1-9.

11. The construct according to claim 10, **characterized in that** the construct also comprises a wild-type Mmup monomer.

12. The construct according to claim 10 or 11, **characterized in that** the construct comprises 1 to 20 Mmup monomer variants, wherein the Mmup monomer variants are identical or different.

13. The construct according to any one of claims 10-12, **characterized in that** the construct comprises 1 to 20 wild-type Mmup monomers.

14. The construct according to any one of claims 10-13, **characterized in that** the Mmup monomer variant and the Mmup monomer variant, the wild-type Mmup monomer and the wild-type Mmup monomer, as well as the Mmup monomer variant and the wild-type Mmup monomer are covalently attached.

15. The construct according to any one of claims 10-14, **characterized in that** the construct comprises 4 to 10 identical or different Mmup monomer variants.

16. A porin comprising at least one Mmup monomer variant, **characterized in that** the Mmup monomer variant comprises an amino acid sequence with any one or more amino acid mutations at 91st to 99th sites of SEQ ID NO: 1, the mutations lead to make a difference in electrical resistance within the pore due to the difference in the physical or chemical properties of different kinds of nucleotides when a single polynucleotide strand passes through the porin comprising at least one Mmup monomer variant.

17. The porin comprising at least one Mmup monomer variant according to claim 16, **characterized in that** the Mmup monomer variant comprises mutations of aspartic (D) at 91^{st} site and/or alanine (A) at 99^{th} site.

18. The porin comprising at least one Mmup monomer variant according to claim 16 or 17, **characterized in that** the Mmup monomer variant comprises at least one of the following mutations:
Aspartic (D) at 91st site is mutated to: proline (P), tryptophan (W), arginine (R), glutamine (Q), lysine (K), phenylalanine (F), serine (S ), asparagine (N), cysteine (C), isoleucine (I), leucine (L) or valine (V), or non-natural amino acid;or,
Alanine (A) at 99th site is mutated to: proline (P), phenylalanine (F), isoleucine (I), leucine (L), valine (V), lysine (K) or arginine (R), or, non-natural amino acid.

19. The porin comprising at least one Mmup monomer variant according to any one of claims 16-18, **characterized in that** the Mmup monomer variant comprises mutations of D91K and/or A99K.

20. The porin comprising at least one Mmup monomer variant according to any one of claims 16-19, **characterized in that** the Mmup monomer variant comprises mutations of D91K and A99K.

21. The porin comprising at least one Mmup monomer variant according to any one of claims 16-20, **characterized in that** the Mmup monomer variant also comprises an amino acid sequence with any one or more amino acid mutations at 80^{th} to 90^{th} sites and/or 100^{th} to 120^{th} sites of SEQ ID NO: 1.

22. The porin comprising at least one Mmup monomer variant according to any one of claims 16-21, **characterized in that** the Mmup monomer variant also comprises an amino acid sequence with any one or more amino acid mutations at 1^{st} to 79^{th} sites and/or 121^{st} to 186^{th} sites of SEQ ID NO: 1.

23. The porin comprising at least one Mmup monomer variant according to any one of claims 16-22, **characterized in that** the Mmup monomer variant alsocomprises one or a combination of two or more of a mutation of leucine (L) at 89^{th} site, a mutation of asparagine (N) at 110^{th} site, a mutation of aspartic (D) at 120^{th} site, a mutation of asparagine (N) at 136^{th} site or a mutation of serine (S) at 141^{st} site.

24. The porin comprising at least one Mmup monomer variant according to any one of claims 16-23, **characterized in that** the Mmup monomer variant comprises at least one of the following mutations:
Leucine (L) at 89^{th} site is mutated to: asparagine (N), alanine (A), glutamine (Q), glycine (G), serine (S), threonine (T), lysine(K) or proline (P), or, non-natural amino acid; or,
Asparagine (N) at 110^{th} site is mutated to: proline (P), alanine (A), isoleucine (I) or leucine (L), or non-natural amino acid; or,
Aspartic (D) at 120^{th} site is mutated to: arginine ^{®}, lysine (K), asparagine (N), glutamine (Q), alanine (A), serine (S), glycine(G) or threonine (T), or non-natural amino acid; or,
Asparagine (N) at136th site is mutated to: arginine ^{®}, lysine (K), glutamine (Q), alanine (A), serine (S) or threonine (T), or non-natural amino acid; or,
Serine (S) at 141^{st} site is mutated to: arginine ^{®}, lysine (K), glutamine (Q), alanine (A), serine (S) or threonine (T), or non-natural amino acid.

25. The porin comprising at least one Mmup monomer variant according to any one of claims 16-24, **characterized in that** the porin comprising at least one Mmup monomer variant comprises at least 1 to 20 Mmup monomer variants, wherein the Mmup monomer variants are identical or different.

26. The porin comprising at least one Mmup monomer variant according to any one of claims 16-25, **characterized in that** the porin comprising at least one Mmup monomer variant comprises the wild-type Mmup monomer.

27. The porin comprising at least one Mmup monomer variant according to any one of claims 16-26, **characterized in that** the porin comprising at least one Mmup monomer variant comprises 1 to 20 wild-type Mmup monomers.

28. The porin comprising at least one Mmup monomer variant according to any one of claims 16-27, **characterized in that** the porin comprising at least one Mmup monomer variant comprises 4 to 10 identical or different Mmup monomer variants.

29. The porin comprising at least one Mmup monomer variant according to any one of claims 16-28, **characterized in that** the Mmup monomer variant and the Mmup monomer variant, the wild-type Mmup monomer and the wild-type Mmup monomer, as well as the Mmup monomer variant and the wild-type Mmup monomer are covalently attached.

30. The porin comprising at least one Mmup monomer variant according to any one of claims 16-29, **characterized in that** the porin comprising at least one Mmup monomer variant comprises a constriction and a loops region.

31. The porin comprising at least one Mmup monomer variant according to claim 30, **characterized in that** the pore channel diameter of the constriction of the porin comprising at least one Mmup monomer variant is less than the pore channel diameter of the constriction of the porin comprising the wild-type Mmup monomer.

32. The porin comprising at least one Mmup monomer variant according to claim 30 or 31, **characterized in that** the pore channel diameter of the constriction of the porin comprising at least one Mmup monomer variant is less than 15.5Å, preferably, the pore channel diameter of the constrictionof the porin comprising at least one Mmup monomer variant is less than13.5Å.

33. The porin comprising at least one Mmup monomer variant according to any one of claims 30-32, **characterized in that** the porin comprising at least one Mmup monomer variant also comprises a cap forming region and/or a barrel forming region.

34. A nucleotide sequence encoding the Mmup monomer variant of any one of claims 1-9, the construct of any one of claims 10-15, or the porin of any one of claims 16-33 comprising at least one Mmup monomer variant.

35. A vector comprising the nucleotide sequence of claim 34.

36. The vector according to claim 35, **characterized in that** the vector comprises a promoter operably linked with the nucleotide sequence encoding the Mmup monomer variant of any one of claims 1-9, the construct of any one of claims 10-15, or the porin of any one of claims 16-33 comprising at least one Mmup monomer variant.

37. The vector according to claim 36, **characterized in that** the promoter is an inducible promoter or a constitutive promoter, wherein the inducible promoter is an acetamide inducible promoter.

38. The vector according to any one of claims 35-37, **characterized in that** the nucleotide sequence encoding the porin comprising at least one Mmup monomer variant comprises at least one nucleotide sequence encoding the Mmup monomer variant.

39. The vector according to claim 38, **characterized in that** the nucleotide sequence encoding the porin comprising at least one Mmup monomer variant also comprises at least one nucleotide sequence encoding the wild-type Mmup monomer.

40. The vector according to claim 38 or 39, **characterized in that** the nucleotide sequence encoding the Mmup monomer variant and the nucleotide sequence encoding the Mmup monomer variant, the nucleotide sequence encoding the Mmup monomer variant and the nucleotide sequence encoding the wild-type Mmup monomer, or, the nucleotide sequence encoding the wild-type Mmup monomer and the nucleotide sequence encoding the wild-type Mmup monomer are linked by sequences encoding amino acid linker.

41. A mutant bacterium expressing the Mmup monomer variants of any one of claims 1-9, the construct of any one of claims 10-15, or the porin comprising at least one Mmup monomer variant of any one of claims 16-33, **characterized in that**, the bacterium comprises:
(a)The deletion of the wild-type Mmup monomer; and (b) The vector of any one of claims 35-40.

42. The mutant bacterium according to claim 41, **characterized in that** the bacterium is *Mycobacterium mucinae.*

43. A method of producing the Mmup porin, **characterized in that** the method includes: transforming the bacterium of any one of claims 41-42 with vector of any one of claims 35-40, and inducing the bacterium to express the Mmup porin.

44. A method of characterizing a target polynucleotide, **characterized in that** method includes:
(a) Contacting the target polynucleotide with porin of any one of claims 16-33 comprising at least one Mmup monomer variant, so that the target polynucleotide sequence passes through the porin comprising at least one Mmup monomer variant; and
(b) Obtaining one or more characteristics of the interaction between the nucleotide and the porin comprising at least one Mmup monomer variant when the target polynucleotide passes through the porin comprising at least one Mmup monomer variant to characterize the target polynucleotide.

45. The method according to claim 44, **characterized in that** the step (a) also includes a step of contacting the target polynucleotide with one or a combination of two or more of the nucleic acid binding protein, helicase, exonuclease, telomerase, topoisomerase, reverse transcriptase, translocase and/or the polymerases, which makes the translocation speed of the target polynucleotide sequence through the porin less than the translocation speed in the absence of the nucleic acid binding protein, helicase, exonuclease, telomerase, topoisomerase, reverse transcriptase, translocase, and/or polymerase.

46. The method according to claim 44 or 45, **characterized in that** the method also includes a step of applying a potential difference across the porin contacted with the target polynucleotide.

47. An application in characterizing the target polynucleotide of the porin of any one of claims 16-33 comprising at least one Mmup monomer variant, the Mmup monomer variant of any one of claims 1-9, the construct of any one of claims 10-15, the nucleotide sequence of claim 34, the vector of any one of claims 35-40, or the mutant bacterium of any one of claims 41-42.

48. A kit for characterizing the target polynucleotide, **characterized in that** the kit comprises porin of any one of claims 16-33 comprising at least one Mmup monomer variant, the Mmup monomer variant of any one of claims 1-9, theconstruct of any one of claims 10-15, the nucleotide sequence of claim 34, the vector of any one of claims 35-40, or the mutant bacterium of any one of claims 41-42.

49. A device for characterizing the target polynucleotide, **characterized in that** the device comprises the porin of any one of claims 16-33 comprising at least one Mmup monomer variant, the Mmup monomer variant of any one of claims 1-9, the construct of any one of claims 10-15, the nucleotide sequence of claim 34, the vector of any one of claims 35-40, or the mutant bacterium of any one of claims 41-42.
